# EUROPEAN PATENT SPECIFICATION

(11) **EP 3 620 166 B1**
(45) Date of publication and mention of the grant of the patent: **10.11.2021**
(21) Application number: 19168464.6
(22) Date of filing: 10.04.2019
(51) Int. Cl.: A61K 36/02, A61P 3/04, A61K 9/14, A61K 47/02, A61K 47/20, A61K 9/00

(54) **COMPOSITION FOR ANTI-OBESITY COMPRISING THE PULVERIZED MATERIAL OF ODONTELLA AURITA AS AN ACTIVE INGREDIENT**
ZUSAMMENSETZUNG GEGEN ADIPOSITAS MIT PULVERFÖRMIGEM MATERIAL VON ODONTELLA AURITA ALS WIRKSTOFF
COMPOSITION ANTI-OBÉSITÉ COMPRENANT LA MATIÈRE PULVÉRISÉE D'ODONTELLA AURITA COMME INGRÉDIENT ACTIF

(30) Priority: 10.09.2018 KR 20180107895
(43) Date of publication of application: 11.03.2020
(73) Proprietor: Korea Institute of Science and Technology, Seoul 02792 (KR)
(72) Inventor: PAN, Cheol-Ho, 25451 Gangwon-do (KR); KIM, Sang Min, 25451 Gangwon-do (KR); KOO, Song Yi, 25451 Gangwon-do (KR); CHA, Kwang-Hyun, 25451 Gangwon-do (KR); LEE, Eun Ha, 25451 Gangwon-do (KR); LEE, Hee Ju, 25451 Gangwon-do (KR); YANG, Jung-Seok, 25451 Gangwon-do (KR); CHOI, Yongsoo, 25451 Gangwon-do (KR); KANG, Kyungsu, 25451 Gangwon-do (KR); KIM, Hyoung Seok, 25451 Gangwon-do (KR); SONG, Dae-geun, 25451 Gangwon-do (KR)
(74) Representative: advotec.

(56) References cited:
- FR-A1- 2 795 959
- KR-B1- 101 855 837
- "Caféin'Algue Intensive fat Burner", GNPD, 1 November 2007 (2007-11-01), XP002681091, [retrieved on 2007-11-01]
- HAMZA AMINE ET AL: "Odontella aurita-enriched diet prevents high fat diet-induced liver insulin resistance", JOURNAL OF ENDOCRINOLOGY, vol. 228, no. 1, 12 October 2015 (2015-10-12), pages 1-12, XP55637610, GB ISSN: 0022-0795, DOI: 10.1530/JOE-15-0316
- ADIL HAIMEUR ET AL: "The role of Odontella aurita, a marine diatom rich in EPA, as a dietary supplement in dyslipidemia, platelet function and oxidative stress in high-fat fed rats", LIPIDS IN HEALTH AND DISEASE, BIOMED CENTRAL, LONDON, GB, vol. 11, no. 1, 31 October 2012 (2012-10-31), page 147, XP021136378, ISSN: 1476-511X, DOI: 10.1186/1476-511X-11-147

## Description

### BACKGROUND OF THE INVENTION

### Field of the Invention

Disclosed are a composition for anti-obesity comprising a pulverized material of *Odontella aurita* as an active ingredient and a method of processing *Odontella aurita* to improve the anti-obesity effects.

### [Description about National Support Research and Development]

This research has been carried out under the supervision of the Korea Institute of Science and Technology with the support of the Ministry of Oceans and Fisheries (specialized organization for research management: Korea Institute of Marine Science & Technology Promotion, title of research project: Development and industrialization of high value added cosmetic raw materials from marine microalgae, project assignment number: 1525003676).

### Description of the Related Art

Obesity refers to an excess of body fat and can be defined as a health risk condition that cause many diseases including heart diseases stemming from excessive body fat. The World Health Organization (WHO) reported that at least about 106 million adults worldwide are overweight, with at least 4 million obese and at least two out of three Americans overweight or obese (Low et al, 2009; Cooke and Bloom, 2006). Also, at least about 20% of the adults in the UK have been diagnosed with obesity. The rate of obesity in Korea has been increasing very rapidly from 13.9% in 1995 to 30.6% in 2001 (The Korean Society for the Study of Obesity, 2010). It is known that obese people are at increased risk for a variety of diseases, including type 2 diabetes, hyperlipidemia, arthritis, and apnea, compared with people who have normal weight, and that in particular, obesity causes a variety of cancers and heart diseases. The National Institutes of Health (NIH) reported that 20 percent of deaths were caused by obesity, which was more than the rate of deaths from tobacco (Shi and Burn, 2006).

It is difficult to conclude that overweight and obesity originate from a single cause. However, they are basically caused by taking in more calories than that consumed by our body (NIH, 2010). The causes of obesity are mainly classified into endocrine factors, genetic factors, and social environmental factors. However, the percentage of obesity attributed to secondary factors such as endocrine diseases or genetic factors is less than 5%. Also, it can be said that most obesity is simple obesity that does not stem from a pathological cause, and that it is caused by the eating habits of taking in more calories than that consumed (The Korean Society for the Study of Obesity, 2010).

Because most obesity and overweight are attributed to excessive calorie intake and lack of physical activity, improving lifestyle through diet and exercise is the basis of the treatment of obesity. In the treatment of obesity, weight loss and waist circumference reduction are important goals. While the principle of the treatment is simple, it is very difficult to achieve long-term weight loss. Therefore, obesity should be treated as a chronic disease requiring continuous treatment for at least several years. Exercise therapy and behavior therapy are used in combination with low calorie diet for improvement of lifestyle, and surgical operation is used for some severe obesity.

Drug-based treatments for obesity are mainly classified into three approaches (Cooke and Bloom, 2006; Shi and Burn, 2004). The first approach is to suppress appetite. This method stimulates the brain's nerves to give a feeling of fullness. Diethylproprion, phendimetrazine, phentermine, etc. have been approved by the Food and Drug Administration (FDA) and used for the treatment of obesity. The second approach is to inhibit the absorption of ingested fat. A representative drug is Orlistat. This drug acts on the digestive organs, prevents the absorption of fat and can be used for a long time. The third approach is to induce weight loss through high energy consumption in vivo. A representative drug is sibutramine, which increases energy consumption through neuromodulation. However, these medications cause nervine side effects or side effects such as inhibition of absorption of fat-soluble vitamins and thus should be used with caution. Especially, obesity medications are not recommended for children, pregnant women or people with diseases such as hypertension. Most obesity requires long-term treatment because after a short period of weight loss, weight regain is experienced. Thus, there is a need for developing a new substitute.

Currently, attempts are being made worldwide to find a composition that prevents or treats obesity and overweight. Representative researches include isolation of natural compositions from plants. In Northeast Asia including Korea, researches to find a composition for preventing or treating obesity and overweight from natural products has been expanding from medicinal plants to wild herbs and vegetables, vegetables and fruits. The present inventors have also made efforts to develop a material for preventing or treating obesity and overweight from plants which are not known at all. Among the plants, the inventors paid attention to microalgae, which are classified as plants and capable of producing biomass throughout the year through culture.

XP002681091 "Caféin Algue Intensive fat Burner", GNPD, 1 November 2007 (2007-00-01), [retrieved on 2007-11-01] ^{∗}page 2^{∗} discloses the use of odontella aurita powder in a composition for non-therapeutic for slimming.

HAMZA AMINE ET AL: "Odontella aurita-enriched diet prevents high fat diet-induced liver insulin resistance", JOURNAL OF EDOCRINOLOGY, vo. 228, no. 1. 12 October 2015 (2015-10-12), pages 1-12, XP55637610, GB, ISSN: 0022-0795, D01: 10.1530/JOE-15-0316 discloses that freeze-dried odontella aurita reduces the body weight of rats fed a high fat diet.

### Citation List

### Patent Literature

Patent Literature 1: Korean Patent Laid-Open No. 10-2014-0033490

### SUMMARY OF THE INVENTION

In one aspect, the present disclosure is to provide a composition for anti-obesity which can be ingested continuously without human toxicity.

In another aspect, the present disclosure is to provide a method of processing *Odontella aurita* to improve the anti-obesity effects.

In one aspect, the technology disclosed herein provides a pulverized material of *Odontella aurita* for anti-obesity.

In one exemplary embodiment, the pulverized material of *Odontella aurita* may reduce body weight.

In one exemplary embodiment, the pulverized material of *Odontella aurita* may inhibit fat accumulation.

The pulverized material of the present invention is a microfluidized material of *Odontella aurita.*

In one exemplary embodiment, the pulverized material may be a lyophilisate of the microfluidized material of *Odontella aurita.*

Further described herein is a non-therapeutic use of the pulverized material of *Odontella aurita* for anti-obesity.

In still another aspect, the technology disclosed herein provides a method of processing *Odontella aurita* to improve the anti-obesity effects comprising the steps of: preparing a suspension comprising *Odontella Aurita*; and injecting the suspension into a microfluidizer to obtain a microfluidized material.

In one exemplary embodiment, the method may further comprise the step of freeze drying the microfluidized material.

In one exemplary embodiment, the suspension may comprise water as a solvent.

In one exemplary embodiment, the suspension may comprise *Odontella aurita* at a concentration of 10³ to 10⁸ cells/ml.

In one exemplary embodiment, the processing pressure of the microfluidizer may be 20.7 to 137.9 MPa (3000 to 20000 psi).

In one exemplary embodiment, the processing by the microfluidizer may be performed once.

In one exemplary embodiment, the freeze drying may be freezing the microfluidized material at -80°C or less and then reducing the pressure 0.13 to 66.7 Pa (1 to 500 mTorr) to sublimate and remove ice and prepare a dried material.

In one aspect, the technology disclosed herein has the effect of providing a composition for anti-obesity comprising the pulverized material of *Odontella aurita* as an active ingredient.

In another aspect, the technology disclosed herein has the effect of providing a method of processing *Odontella aurita* that improves the anti-obesity effects.

### BRIEF DESCRIPTION OF THE DRAWINGS

FIG. 1 shows the results of measuring the food efficiency ratio of a test group according to one test example of the present disclosure;
FIG. 2 shows the results of measuring the epididymal fat weight of a test group according to one test example of the present disclosure; and
FIG. 3 shows the results of measuring the total content of the carotenoids eluted from the lyophilisate of *Odontella aurita* and the lyophilisate of the microfluidized material of *Odontella aurita* according to one example of the present disclosure.

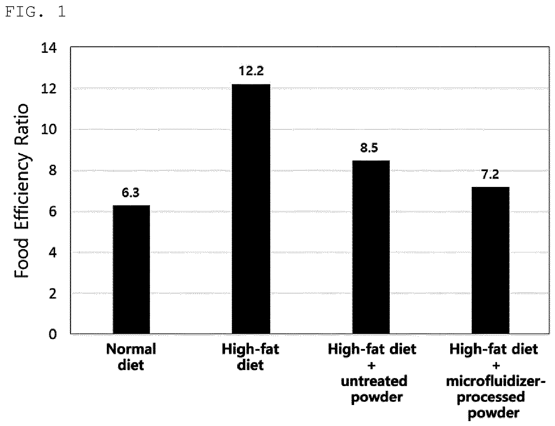

In the graphs of FIG. 1 to FIG. 3, "untreated" and "untreated powder" denote the lyophilisate of Example 1, which is a test group not processed with a microfluidizer, and "microfluidizer-processed" and "microfluidizer-processed powder" denote the lyophilisate of Example 2, which is a test group processed with a microfluidizer.

### DETAILED DESCRIPTION OF THE PREFERRED EMBODIMENTS

Hereinafter, the present invention as defined in the claims will be described in detail.

In one aspect, the technology disclosed herein provides a composition for anti-obesity comprising the pulverized material of *Odontella aurita* as an active ingredient.

In another aspect, the technology disclosed herein provides the pulverized material of *Odontella aurita* for anti-obesity purposes.

*Odontella aurita* is a diatom belonging to species *Odontella* belonging to the family Eupodiscaceae of the order Centrales and is unicellular marine microalgae. It is about 50 µm in size, has a rectangular valve shape, and has two or more labiate processes in each valve. The cell has a large number of chlorophylls, and each cell may be connected in a straight line to form a straight chain. *Odontella aurita* is present all over the world including Europe, the Americas, Asia, Australia and New Zealand, and is found all over the coast of Korea. *Odontella aurita* is not toxic to humans and thus can be ingested continuously.

As used herein, *Odontella aurita* is not limited in the method of obtaining it, whether it was collected, cultured, or marketed.

As used herein, the term "active ingredient" refers to an ingredient that exhibits the desired activity by itself or an ingredient which can exhibit the desired activity together with a carrier which itself has no activity, etc.

As used herein, the term "anti-obesity" refers to preventing, inhibiting, ameliorating, alleviating, or treating obesity. Obesity means a state in which excess fat has accumulated in the body. When energy intake is higher than energy consumption, the excess energy remaining accumulates to form body fat in the form of various lipids or increases the lipid content in the blood, and this state is called obesity.

In one exemplary embodiment, the composition or the pulverized material of *Odontella aurita* may reduce body weight.

In one exemplary embodiment, the composition or the pulverized material of *Odontella aurita* may inhibit weight gain.

In one exemplary embodiment, the composition or the pulverized material of *Odontella aurita* may inhibit fat accumulation.

In one exemplary embodiment, the composition or the pulverized material of *Odontella aurita* may inhibit fat accumulation in the epididymis.

In one exemplary embodiment, the composition or the pulverized material of *Odontella aurita* may inhibit body fat accumulation resulting from a high-fat diet.

In one exemplary embodiment, the pulverized material may be the pulverized material of *Odontella aurita* cells. The pulverized material may be in the form of a suspension or a powder.

The pulverized material is a microfluidized material of *Odontella aurita*, i.e., a microfluidizer-processed pulverized material obtained by processing with a microfluidizer. The microfluidization refers to preparing a physically stable suspension in which particles of relatively consistent size are well dispersed, by using a microfluidizer. The microfluidized material may be obtained using a microfluidizer.

In one exemplary embodiment, the pulverized material may be a dried material of the pulverized material of *Odontella aurita.* The drying method for preparing the dried material may be any conventional drying method in the art. Specifically, it may be a conventional drying method in the art such as freeze drying, spray drying, and hot air drying. A dried powder of the pulverized material of *Odontella aurita* can be obtained by these methods.

In one exemplary embodiment, the pulverized material may be a dried material, for example, lyophilisate, of the microfluidized material of *Odontella aurita.* The freeze drying, which is a type of drying method, refers to making dried materials by freezing materials and then subjecting the resultant materials to a sublimation process of directly making ice into water vapor by lowering the partial pressure of water vapor. For example, a freeze-dried sample may be obtained by freezing a material at a temperature of -80°C or less using a freezer and then reducing the pressure to sublimate ice and remove water.

In one exemplary embodiment, the pulverized material may be a lyophilisate of the microfluidized material of *Odontella aurita* obtained by subjecting a suspension comprising *Odontella aurita* cells to a microfluidizer and a freeze dryer.

In one exemplary embodiment, the pulverized material of *Odontella aurita* may be applied or administered to a subject in the form of a composition, for example, a pharmaceutical composition or a food composition.

In one exemplary embodiment, the content of the pulverized material of *Odontella aurita* may be 0.01 to 50% by weight based on the total weight of the composition. Specifically, the content of the pulverized material of *Odontella aurita* may be 0.01% by weight or more, 0.05% by weight or more, 0.1% by weight or more, 0.5% by weight or more, 1% by weight or more, 5% by weight or more, 10% by weight or more, 20% by weight or more, 30% by weight or more, or 40% by weight or more and 50% by weight or less, 40% by weight or less, 30% by weight or less, 20% by weight or less, 10% by weight or less, 5% by weight or less, 1% by weight or less, 0.5% by weight or less, 0.1% by weight or less, or 0.05% by weight or less, based on the total weight of the composition.

In one exemplary embodiment, the composition may be a pharmaceutical composition or a food composition.

In one exemplary embodiment, the composition may be a health functional food composition.

The pharmaceutical composition according to one aspect of the present disclosure may be various oral or parenteral formulations. When the composition is formulated, generally used diluents or excipients such as fillers, extenders, binders, humectants, disintegrants, surfactants are used. Solid preparations for oral administration include tablets, pills, powders, granules, soft or hard capsules, etc., and are prepared by mixing at least one compound with at least one excipient, for example, starch, calcium carbonate, sucrose or lactose, gelatin, etc. Further, lubricants such as magnesium stearate, talc, etc. are used in addition to simple excipients. Liquid preparations for oral administration include suspensions, liquids for internal use, emulsions, syrups, etc., and various excipients such as humectants, sweeteners, flavoring agents, preservatives, etc. as well as water and liquid paraffin, which are generally used simple diluents, may be added. Formulations for parenteral administration include sterile aqueous solutions, non-aqueous solvents, suspensions, emulsions, freeze-dried formulations, and suppositories. Examples of the non-aqueous solvents and the suspension solvents include propylene glycol, polyethylene glycol, vegetable oil such as olive oil, injectable esters such as ethyl oleate. Examples of the matrix for suppositories include witepsol, macrogol, tween 61, cacao butter, laurinum, glycerogelatin, etc.

The pharmaceutical dosage form of the composition according to one aspect of the present disclosure may be in the form of its pharmaceutically acceptable salts. In addition, the pharmaceutical dosage form of the composition may be used alone or as a combination or an appropriate assembly with other pharmaceutically active compounds. The salt is not specifically limited as long as it is pharmaceutically acceptable. For example, hydrochloric acid, sulfuric acid, nitric acid, phosphoric acid, hydrofluoric acid, hydrobromic acid, formic acid, acetic acid, tartaric acid, lactic acid, citric acid, fumaric acid, maleic acid, succinic acid, methanesulfonic acid, benzenesulfonic acid, toluenesulfonic acid, naphthalenesulfonic acid, etc. may be used.

The composition according to one aspect of the present disclosure may be administered parenterally or orally, according to the purpose. It may be administered in one to several divided doses so that the daily dose is 0.1-1,500 mg, preferably 1-500 mg, per 1 kg of body weight. The dosage for a patient may vary depending on the patient's body weight, age, gender, health condition, diet, time of administration, administration method, excretion rate, severity of disease, etc.

The pharmaceutical composition according to one aspect of the present disclosure may be formulated into any form suitable for pharmaceutical preparations, including oral formulations such as powders, granules, tablets, soft or hard capsules, suspensions, emulsions, syrups, drinks and aerosols, agents for external application to the skin such as ointments and creams, suppositories, injections, and sterile injection solutions.

The composition for the use according to one aspect of the present invention may be administered to mammals such as rats, mice, livestock, humans, etc. through various routes such as parenteral, oral, etc., and all of the administration methods are predictable. For example, it may be administered orally, transdermally, rectally, intravenously, intramuscularly, or through subcutaneous, intrauterine dura mater or intracerebroventricular injection.

The composition for the use according to one aspect the present invention may be administered via various routes that can be easily applied by those skilled in the art.

The formulation of the food composition for the use according to one aspect of the present invention is not particularly limited, but the composition may be formulated into, for example, liquid formulations such as tablets, granules, powders and drinks, caramels, gels, bars, etc. In the food composition of each formulation, other ingredients commonly used in the art may be properly selected and added by those skilled in the art without any difficulty, in addition to the active ingredient, according to the formulation or purpose. The addition of other ingredients may give a synergic effect.

The pulverized material of *Odontella aurita* for the use according to one aspect of the present invention may be added to health supplements or health functional foods such as foods and beverages, for the purpose of weight loss and prevention or amelioration of obesity. When the pulverized material of *Odontella aurita* is used as a food additive, it may be added in an amount of 0.01 to 20% by weight, preferably 0.1 to 5% by weight, based on the total weight of the food. The amount of the active ingredient, *Odontella aurita*, may be appropriately determined according to the intended use (prevention, health or therapeutic treatment). However, in the case of long-term intake for the purpose of health and hygiene or for the purpose of controlling health conditions, the amount may be less than the above range. Also, the active ingredient may be used in an amount exceeding the above range, since it has no problem with stability. The powder may be used in combination with other foods or food ingredients, and may be appropriately used according to conventional methods.

In the food composition for the use according to one aspect of the present invention, the determination of the dose of the active ingredient is within the knowledge of those skilled in the art. The daily dose of the active ingredient may be, for example, 0.1 mg/kg/day to 5000 mg/kg/day, more specifically, 50 mg/kg/day to 500 mg/kg/day, although not limited thereto. Also, it may vary depending on various factors such as the age, health condition, complications, etc. of the subject.

The food composition for the use according to one aspect of the present invention may be, for example, various foods such as chewing gums, caramel products, candies, frozen desserts, and confectionery, beverage products such as soft drinks, mineral water, and alcoholic beverages, and health functional foods such as vitamins and minerals.

In addition to the above, the food composition for the use of the present invention may contain various nutrients, vitamins, minerals (electrolytes), flavors such as synthetic flavors and natural flavors, coloring agents, enhancers (cheese, chocolate, etc.), pectic acid and its salts, alginic acid and its salts, organic acids, protective colloidal thickeners, pH regulators, stabilizers, antiseptics, glycerin, alcohols, and carbonators used for carbonated drinks, etc. The functional food composition for the use of the present invention may also contain natural fruit juice and fruit flesh for preparation of fruit beverages and vegetable beverages. These ingredients may be used alone or as a mixture. The proportion of these additives is not critical, but in general, the content of these additives is about 0 to 20 parts by weight based on 100 parts by weight of the composition of the present disclosure.

In another aspect, the technology disclosed herein provides a method of processing *Odontella aurita* to improve the anti-obesity effects comprising the steps of: preparing a suspension comprising *Odontella Aurita*; and injecting the suspension into a microfluidizer to obtain a microfluidized material.

In one exemplary embodiment, the present invention may further comprise the step of drying, for example, freeze drying the microfluidized material.

In one exemplary embodiment, the *Odontella Aurita* may be prepared by centrifuging cultured *Odontella aurita* and collecting the cells.

In one exemplary embodiment, the centrifugation may be performed at 5,000 rpm or more, 7,000 rpm or more, 9,000 rpm or more, 10,000 rpm or more, 13,000 rpm or more, 15,000 rpm or more, 17,000 rpm or more, or 20,000 rpm or more or 20,000 rpm or less, 17,000 rpm or less, 15,000 rpm or less, 13,000 rpm or less, 10,000 rpm or less, 9,000 rpm or less, 7,000 rpm or less, or 5,000 rpm or less.

In one exemplary embodiment, the centrifugation may be performed at a rate of 100 L or more, 200 L or more, 300 L or more, 400 L or more, 500 L or more, 600 L or more, 700 L or more, 800 L or more, 900 L or more, or 1,000 L or more per hour or 1,000 L or less, 900 L or less, 800 L or less, 700 L or less, 600 L or less, 500 L or less, 400 L or less, 300 L or less, 200 L or less, or 100 L or less per hour.

In one exemplary embodiment, the *Odontella aurita* may be cultured in a photobioreactor.

In one exemplary embodiment, the *Odontella aurita* may be cultured for a period of 1 day or more, 3 days or more, 5 days or more, 7 days or more, 9 days or more, 11 days or more, or 13 days or more or 15 days or less, 13 days or less, 11 days or less, 9 days or less, 7 days or less, 5 days or less, 3 days or less, or 1 day or less.

In one exemplary embodiment, the suspension may comprise water as a solvent.

In one exemplary embodiment, a suspension comprising *Odontella aurita* at a concentration of 10³ to 10⁸ cells/ml may be preferable for improving the anti-obesity efficacy of *Odontella aurita.* The concentration of the suspension acts as a major factor in enhancing the anti-obesity efficacy of *Odontella aurita.* In another exemplary embodiment, the suspension comprises *Odontella aurita* at a concentration of 10³ cells/ml or more, 10⁴ cells/ml or more, 10⁵ cells/ml or more, 10⁶ cells/ml or more or 10⁷ cells/ml or more and 10⁸ cells/ml or less and thereby prevents the problems that clogging of the nozzle of the microfluidizer makes the microfluidization process not smooth and that the microfluidized material may have an insignificant effect in enhancing the anti-obesity efficacy.

In one exemplary embodiment, the processing pressure of the microfluidizer may be 20.7 to 137.9 MPa (3000 to 20.000 psi). In another exemplary embodiment, the processing pressure of the microfluidizer may be 20.7 MPa (3000 psi) or more, 27.6 MPa (4000 psi) or more, 34.5 MPa (5000 psi) or more, 41.4 MPa (6000 psi) or more, 48.3 MPa (7000 psi) or more, 55.2 MPa (8000 psi) or more, 62.1 Mpa (9000 psi) or more or 68.9 MPa (10000 psi) or more and 137.9 MPa (20000 psi) or less, 103.4 MPa (15000 psi) or less, 68.9 MPa (10000 psi) or less or 34.5 MPa (5000 psi) or less. The processing pressure of the microfluidizer is a major factor in enhancing the anti-obesity efficacy of *Odontella aurita.* It is possible to enhance the anti-obesity efficacy of *Odontella aurita* by preparing a suspension in which regular particles are well dispersed, by effectively pulverizing cells with the processing pressure in the above range. In this respect, the processing pressure of the microfluidizer may preferably be between 34.5 and 68.9 MPa (5000 and 10000 psi) or between 20.7 and 48.3 MPa (3000 and 7000 psi).

In one exemplary embodiment, the processing by the microfluidizer may be performed once.

In one exemplary embodiment, the freeze drying may be freezing the microfluidized material at -80°C or less or at -130°C to -80°C and then reducing the pressure to 0.13 to 66.7 Pa (1 to 500 mTorr), 0.12 to 53.3 Pa (1 to 400 mTorr), 0.13 to 40.0 Pa (1 to 300 mTorr), 0.13 to 26.7 Pa (1 to 200 mTorr), 0.13 to 13.3 Pa (1 to 100 mTorr), 1.3 to 10.7 Pa (10 to 80 mTorr), 1.3 to 8.0 Pa (10 to 60 mTorr), 2.7 to 5.4 Pa (20 to 40 mTorr) to sublimate and remove ice and prepare a dried material.

Hereinafter, the present invention will be described in more detail with reference to examples. It will be apparent to those skilled in the art that these examples are for illustrative purposes only, and the scope of the present invention is not construed as being limited by these examples.

### Example 1: Preparation of a lyophilisate of Odontella aurita

*Odontella aurita* (BioOne Co., Ltd., Gangneung, Korea) was cultured in a 400 L photobioreactor for one week using F/2 medium. The thus-cultured *Odontella aurita* was centrifuged at 15,000 rpm at a rate of 500 L per hour using a continuous centrifuge to collect the cells of *Odontella aurita.* The collected cells were freeze-dried (-118°C, 3.73 Pa (28 mTorr)) 2 days, Model FDCF-12003, OPERON, Korea) to obtain lyophilisates of *Odontella aurita.*

### Example 2: Preparation of a microfluidizer-processed pulverized materials of Odontella aurita

*Odontella aurita* was cultured according to the method of Example 1, and the collected *Odontella aurita* cells were suspended in distilled water at a concentration of 10⁸ cells/mL. The resultant suspension was injected into a microfluidizer (Microfluidizer M-110EH, Microfluidics Co., MA, USA) and pulverized. The processing pressure of the microfluidizer for the preparation of the microfluidized material of *Odontella aurita* was 34.5 MPa (5000 psi).

The microfluidized materials obtained by one-time processing with a microfluidizer was freeze-dried (-118°C, 3.73 Pa, 2 days, Model FDCF-12003, OPERON, Korea) to obtain lyophilisates of the microfluidized materials (hereinafter "microfluidizer-processed pulverized materials").

### Test Example 1: Test for the effect of weight loss and inhibition of fat accumulation

In order to investigate the effect of weight loss and inhibition of fat accumulation of the lyophilisate and the microfluidizer-processed pulverized material prepared in Examples 1 and 2, the following test was conducted on C57BL/6 mice (DooYeol Biotech, Seoul, Korea).

First, male C57BL/6 mice were randomly divided into four groups and given a normal diet for one week. Then, the negative control group was given a normal diet, and the high-fat diet group was given a high-fat diet containing 60% by weight of fat for 12 weeks. The test groups were given a high-fat diet containing 10% by weight of the lyophilisate of Example 1 or the microfluidizer-processed pulverized material of Example 2 for 12 weeks. The body weight gain and feed intake of each of the groups fed in these manners were measured, and the results are shown in Table 1 below.

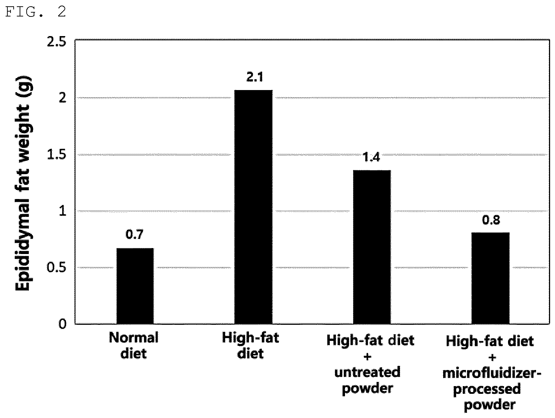

Base on Table 1, the food efficiency ratio (FER, body weight gain/feed intake × 100) of each group was calculated and the results are shown in FIG. 1. In addition, the fat accumulation in the mice of each group was determined by measuring the epididymal fat weight. The results are shown in FIG. 2.

**Table 1**

| | Normal diet | High-fat diet | High-fat diet + lyophilisate of Example 1 | High-fat diet + microfluidizer-processed pulverized material of Example 2 |
|---|---|---|---|---|
| Body weight gain (g) | 12.27 | 24.23 | 15.90 | 14.23 |
| Feed intake (g) | 194.78 | 198.21 | 187.06 | 197.64 |

As shown in Table 1, the high-fat diet test groups fed with the lyophilisate of Example 1 and the microfluidizer-processed pulverized material of Example 2 lost weight by 34.8% and 41.6%, respectively, compared with the high-fat diet group.

In addition, FIG. 1 shows that the high-fat diet test groups fed with the lyophilisate of Example 1 and the microfluidizer-processed pulverized material of Example 2, respectively, exhibited a food efficiency ratio (FER) that is 30.3% and 41.0% lower than the high-fat diet group, which demonstrates that these materials have the effect of inhibiting weight gain.

In addition, FIG. 2 shows that in the high-fat diet test groups fed with the lyophilisate of Example 1 and the microfluidizer-processed pulverized material of Example 2, respectively, the epididymal fat weight decreased by 33.3% and 61.9%, respectively, compared with the high-fat diet group. Thus, it can be understood that lipid accumulation in the epididymis by the high-fat diet was significantly inhibited, and as a result that lipid accumulation did not occur in the epididymis despite the high-fat diet.

In particular, it was found that the test group fed with the lyophilisate of the microfluidized material of *Odontella aurita* obtained by sequentially subjecting a suspension of *Odontella aurita* to a microfluidizer and a freeze dryer showed a remarkably high effect in weight loss and inhibition of fat accumulation. These results show that the pulverized material of *Odontella aurita* according to the present invention, specifically the pulverized material of *Odontella aurita* obtained by microfluidizer processing, has an excellent effect in inhibiting the weight gain due to a high-fat diet and preventing lipid accumulation in the epididymis and thus can be very useful as a safe material for medicines and health functional foods for preventing, ameliorating, or treating obesity.

### Test Example 2: Measurement of the elution degree of a useful material

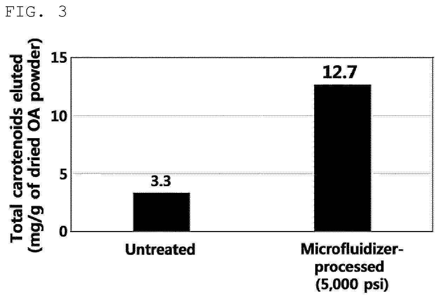

In order to compare the bioavailability at intake of the lyophilisates prepared in Examples 1 and 2, 1 g of each lyophilisate was taken and added with 5 mL of 70% ethanol, and the degree of elution of a useful material during 2 hours was measured. The results are shown in FIG. 3. Carotenoids were selected as the useful material and the content of total carotenoids eluted was measured.

According to the results, the amounts of total carotenoids eluted from 1 g of the lyophilisate of *Odontella Aurita* (OA) (Example 1) and 1 g of the microfluidizer-processed pulverized material of *Odontella aurita* (OA) (Example 2) were 3.3 mg and 12.7 mg, respectively, which shows that the amount of the total carotenoids eluted from the material subjected to microfluidizer processing is 3.8 times higher.

Thus, it can be seen that the microfluidizer-processed pulverized material according to the present disclosure can exhibit increased bioavailability by allowing the useful material to be eluted well during the actual digestion process.

Hereinafter, formulation examples of the composition according to one aspect of the present invention will be described. However, it may be formulated into various other forms. These examples are for illustrative purposes only and are not intended to limit the scope of the present invention.

### [Formulation Example 1] Soft capsule

8 mg of the pulverized material of *Odontella aurita* of Example 2, 9 mg of vitamin E, 9 mg of vitamin C, 2 mg of palm oil, 8 mg of vegetable hydrogenated oil, 4 mg of yellow wax, and 9 mg of lecithin were mixed according to a conventional method to prepare a soft capsule filling liquid. 400 mg of the resultant filling liquid was filled into each capsule to prepare a soft capsule. Separately from the above, a soft capsule sheet was prepared with 66 parts by weight of gelatin, 24 parts by weight of glycerin, and 10 parts by weight of sorbitol solution, and filled with the filling liquid to prepare a soft capsule containing 400 mg of the composition according to the present invention.

### [Formulation Example 2] Tablet

8 mg of the pulverized material of *Odontella aurita* of Example 2, 9 mg of vitamin E, 9 mg of vitamin C, 200 mg of galactooligosaccharide, 60 mg of lactose, and 140 mg of maltose were mixed and granulated into granules using a fluidized bed granulator and added with 6 mg of sugar ester. 500 mg of the resultant composition was tabletted according to a conventional method to prepare a tablet.

### [Formulation Example 3] Drink

8 mg of the pulverized material of *Odontella aurita* of Example 2, 9 mg of vitamin E, 9 mg of vitamin C, 10 g of glucose, 0.6 g of citric acid, and 25 g of liquid oligosaccharide were mixed and added with 300 ml of purified water. 200ml of the resultant mixture was filled into each bottle. After filled into a bottle, the mixture was sterilized at 130°C for 4 to 5 seconds to prepare a drink.

### [Formulation Example 4] Granule

8 mg of the pulverized material of *Odontella aurita* of Example 2, 9 mg of vitamin E, 9 mg of vitamin C, 250 mg of anhydrous crystalline glucose, and 550 mg of starch were mixed and granulated into granules using a fluidized bed granulator, which were then filled in a pouch.

### [Formulation Example 5] Injection

An injection was prepared according to a conventional method with the composition shown in Table 2 below.

**Table 2**

| Ingredient | Content |
|---|---|
| Pulverized material of *Odontella aurita* of Example 2 | 10-50 mg |
| Sterilized distilled water for injection | q.s. |
| pH adjuster | q.s. |

### [Formulation Example 6] Health functional food

A health functional food was prepared according to a conventional method with the composition shown in Table 3 below.

**Table 3**

| Ingredient | Content |
|---|---|
| Pulverized material of *Odontella aurita* of Example 2 | 20 mg |
| Vitamin A acetate | 70 µg |
| Vitamin E | 1.0 mg |
| Vitamin B1 | 0.13 mg |
| Vitamin B2 | 0.15 mg |
| Vitamin B6 | 0.5 mg |
| Vitamin B12 | 0.2 µg |
| Vitamin C | 10 mg |
| Biotin | 10 µg |
| Nicotinamide | 1.7 mg |
| Folic acid | 50 µg |
| Calcium pantothenate | 0.5 mg |
| Ferrous sulfate | 1.75 mg |
| Zinc oxide | 0.82 mg |
| Magnesium carbonate | 25.3 mg |
| Potassium phosphate monobasic | 15 mg |
| Dicalcium phosphate | 55 mg |
| Potassium citrate | 90 mg |
| Calcium carbonate | 100 mg |
| Magnesium chloride | 24.8 mg |

The mixing ratio of the vitamin and mineral mixtures was determined based on the ingredients relatively appropriate for health functional foods, but the mixing ratio may be modified arbitrarily.

### [Formulation Example 7] Health beverage

A health beverage was prepared according to a conventional method with the composition shown in Table 4 below.

**Table 4**

| Ingredient | Content |
|---|---|
| Pulverized material of *Odontella aurita* of Example 2 | 1000mg |
| Citric acid | 1000mg |
| Oligosaccharide | 100 g |
| Taurine | 1g |
| Purified water | Balance |

The above ingredients were mixed according to a conventional method for preparing health beverages. The resultant mixture was heated at 85°C with stirring for about 1 hour. The resultant solution was filtered and sterilized.

### [Formulation Example 8] Flour food

Flour was prepared according to a conventional method with the composition shown in Table 5 below.

**Table 5**

| Ingredient | Content |
|---|---|
| Pulverized material of *Odontella aurita* of Example 2 | 5% by weight |
| Flour | 95% by weight |

Breads, cakes, cookies, crackers and noodles were prepared using the flour prepared in accordance with Table 5, to prepare health-improving foods.

### [Formulation Example 9] Sunsik

Brown rice, barley, glutinous rice, and Coix lacryma-jobi were gelatinized and dried according to a known method, followed by roasting. Then the resultant mixture was prepared into powders having a particle size of 60 mesh using a pulverizer. Black beans, black sesame seeds, and perilla seeds were steamed and dried according to a known method, followed by roasting. The resultant mixture was then prepared into powders having a particle size of 60 mesh using a pulverizer. The above-prepared powders and the pulverized material of *Odontella aurita* of the present disclosure were blended in the ratio of Table 6 below to prepare Sunsik.

**Table 6**

| Ingredient | Content |
|---|---|
| Pulverized material of *Odontella aurita* of Example 2 | 3% by weight |
| Brown rice | 30% by weight |
| Coix lacryma-jobi | 20% by weight |
| Barley | 25% by weight |
| Perilla seeds | 7% by weight |
| Black beans | 7% by weight |
| Black sesame seeds | 7% by weight |
| Glutinous rice | 0.5% by weight |
| Rehmannia glutinosa | 0.5% by weight |

While the present invention has been described with respect to the specific embodiments, it will be apparent to those skilled in the art that the above descriptions are only preferred embodiments and that the scope of the present invention is not limited thereto. Thus, the scope of the present invention should be defined by the appended claims and equivalents thereof.

## Claims

1. A pulverized material of *Odontella aurita* for use for anti-obesity, wherein the pulverized material is a microfluidized material of *Odontella aurita*..

2. The pulverized material of *Odontella aurita* for anti-obesity according to claim 1,
wherein the pulverized material of *Odontella aurita* reduces body weight.

3. The pulverized material of *Odontella aurita* for anti-obesity according to claim 1 or claim 2,
wherein the pulverized material of *Odontella aurita* inhibits fat accumulation.

4. The pulverized material of *Odontella aurita* for anti-obesity according to any one of claims 1 to 3,
wherein the pulverized material is a lyophilisate of the microfluidized material of *Odontella aurita.*

5. A method of processing *Odontella aurita* to improve the anti-obesity effects comprising the steps of:
preparing a suspension comprising *Odontella Aurita*; and
injecting the suspension into a microfluidizer to obtain a microfluidized material.

6. The method of processing *Odontella aurita* to improve the anti-obesity effects according to claim 5,
wherein the method further comprises the step of freeze drying the microfluidized material.

7. The method of processing *Odontella aurita* to improve the anti-obesity effects according to claim 5 or claim 6,
wherein the suspension comprises water as a solvent.

8. The method of processing *Odontella aurita* to improve the anti-obesity effects according to any one of claims 5 to 7,
wherein the suspension comprises *Odontella aurita* at a concentration of 10³ to 10⁸ cells/ml.

9. The method of processing *Odontella aurita* to improve the anti-obesity effects according to any one of claims 5to 8,
wherein the processing pressure of the microfluidizer is 20.7 to 137.9 Mpa (3000 to 20.000 psi).

10. The method of processing *Odontella aurita* to improve the anti-obesity effects according to any one of claims 5to 9,
wherein the processing by the microfluidizer is performed once.

11. The method of processing *Odontella aurita* to improve the anti-obesity effects according to claim 6,
wherein the freeze drying is freezing the microfluidized material at -80°C or less and then reducing the pressure to 0.13 to 66.7 Pa (1 to 500 mTorr) to sublimate and remove ice and prepare a dried material.

## Patentansprüche

1. Pulverisiertes Material aus *Odontella aurita* zur Verwendung gegen Adipositas,
wobei das pulverisierte Material ein mikrofluidisiertes Material aus *Odontella aurita* ist.

2. Pulverisiertes Material aus *Odontella aurita* gegen Adipositas nach Anspruch 1,
wobei das pulverisierte Material aus *Odontella aurita* das Körpergewicht reduziert.

3. Pulverisiertes Material aus *Odontella aurita* gegen Adipositas nach Anspruch 1 oder Anspruch 2,
wobei das pulverisierte Material aus *Odontella aurita* die Fettansammlung hemmt.

4. Pulverisiertes Material aus *Odontella aurita* gegen Adipositas nach einem der Ansprüche 1 bis 3,
wobei das pulverisierte Material ein Lyophilisat des mikrofluidisierten Materials aus *Odontella aurita* ist.

5. Verfahren zur Verarbeitung von *Odontella aurita* zur Verbesserung der Wirksamkeit gegen Adipositas, umfassend die folgenden Schritte:
Herstellen einer Suspension mit *Odontella aurita*; und
Injizieren der Suspension in eine Mikrofluidisierungsvorrichtung zur Gewinnung eines mikrofluidisierten Materials.

6. Verfahren zur Verarbeitung von *Odontella aurita* zu Verbesserung der Wirksamkeit gegen Adipositas nach Anspruch 5,
wobei das Verfahren des Weiteren den Schritt des Gefriertrocknens des mikrofluidisierten Materials umfasst.

7. Verfahren zur Verarbeitung von *Odontella aurita* zur Verbesserung der Wirksamkeit gegen Adipositas nach Anspruch 5 oder Anspruch 6,
wobei die Suspension Wasser als Lösungsmittel umfasst.

8. Verfahren zur Verarbeitung von *Odontella aurita* zur Verbesserung der Wirksamkeit gegen Adipositas nach einem der Ansprüche 5 bis 7,
wobei die Suspension *Odontella aurita* in einer Konzentration von 10³ bis 10⁸ Zellen/ml umfasst.

9. Verfahren zur Verarbeitung von *Odontella aurita* zur Verbesserung der Wirksamkeit gegen Adipositas nach einem der Ansprüche 5 bis 8,
wobei der Verarbeitungsdruck der Mikrofluidisierungsvorrichtung 20,7 bis 137,9 MPa (3000 bis 20 000 psi) beträgt.

10. Verfahren zur Verarbeitung von *Odontella aurita* zur Verbesserung der Wirksamkeit gegen Adipositas nach einem der Ansprüche 5 bis 9,
wobei die Verarbeitung mittels der Mikrofluidisierungsvorrichtung einmal erfolgt.

11. Verfahren zur Verarbeitung von *Odontella aurita* zur Verbesserung der Wirksamkeit gegen Adipositas nach Anspruch 6,
wobei das mikrofluidisierte Material bei dem Gefriertrocknen bei -80 °C oder weniger gefroren und der Druck anschließend auf 0,13 bis 66,7 Pa (1 bis 500 mTorr) verringert wird, um Eis zu sublimieren und zu entfernen und ein getrocknetes Material herzustellen.

## Revendications

1. Matière pulvérisée d'*Odontella aurita* pour l'utilisation contre l'obésité, dans
laquelle la matière pulvérisée est une matière microfluidisée d'*Odontella aurita.*

2. Matière pulvérisée *d'Odontella aurita* contre l'obésité selon la revendication 1,
dans laquelle la matière pulvérisée d'*Odontella aurita* réduit le poids corporel.

3. Matière pulvérisée d'*Odontella aurita* contre l'obésité selon la revendication 1 ou la revendication 2,
dans laquelle la matière pulvérisée d'*Odontella aurita* inhibe l'accumulation de graisse.

4. Matière pulvérisée d'*Odontella aurita* contre l'obésité selon l'une quelconque des revendications 1 à 3,
dans laquelle la matière pulvérisée est un lyophilisat de la matière microfluidisée *d'Odontella aurita.*

5. Procédé de traitement d'*Odontella aurita* pour améliorer les effets anti-obésité comprenant les étapes consistant à :
préparer une suspension comprenant de l'*Odontella aurita* ; et
injecter la suspension à un microfluidiseur afin d'obtenir une matière microfluidisée.

6. Procédé de traitement d' *Odontella aurita* pour améliorer les effets anti-obésité selon la revendication 5,
dans lequel le procédé comprend en outre l'étape consistant à lyophiliser la matière microfluidisée.

7. Procédé de traitement *d'Odontella aurita* pour améliorer les effets anti-obésité selon la revendication 5 ou la revendication 6,
dans lequel la suspension comprend de l'eau comme solvant.

8. Procédé de traitement *d'Odontella aurita* pour améliorer les effets anti-obésité selon l'une quelconque des revendications 5 à 7,
dans lequel la suspension comprend de l'*Odontella aurita* à une concentration de 10³ à 10⁸ cellules/ml.

9. Procédé de traitement d'*Odontella aurita* pour améliorer les effets anti-obésité selon l'une quelconque des revendications 5 à 8,
dans lequel la pression de traitement du microfluidiseur est de 20,7 à 137,9 MPa (3000 à 20 000 psi).

10. Procédé de traitement d'*Odontella aurita* pour améliorer les effets anti-obésité selon l'une quelconque des revendications 5 à 9,
dans lequel le traitement par le microfluidiseur est effectué une fois.

11. Procédé de traitement d'*Odontella aurita* pour améliorer les effets anti-obésité selon la revendication 6,
dans lequel la lyophilisation consiste en congeler la matière microfluidisée à -80 °C ou moins et ensuite réduire la pression à de 0,13 à 66,7 Pa (1 à 500 mTorr) afin de sublimer et éliminer de la glace et préparer une matière séchée.
